# EUROPEAN PATENT APPLICATION

(11) **EP 4 091 677 A1**
(43) Date of publication of application: **23.11.2022**
(21) Application number: 22162887.8
(22) Date of filing: 18.03.2022
(51) Int. Cl.: A61Q 19/10, A61K 8/02, A61K 8/46, A61K 8/365, A61K 8/44, A61K 8/19, A61K 8/34, A61K 8/49, A61K 8/368

(54) **DILUTABLE SOLID FOAMING CLEANSER**

(30) Priority: 18.05.2021 US 202117302985
(71) Applicant: Henkel AG & Co. KGaA, 40589 Düsseldorf (DE)
(72) Inventor: Macary, Jennifer Sullivan, Danbury, 06810 (US)
(74) Representative: Henkel IP Department

(57) **Abstract**

Solid concentrated compositions of foaming cleansers, methods for preparing such solid concentrated compositions, and methods for forming liquid cleansing solutions from such solid concentrated compositions are provided. An exemplary solid concentrated composition includes a surfactant and a dissolution promotion agent, wherein the composition is anhydrous, and wherein the composition is substantially free of sulfates.

## Description

### TECHNICAL FIELD

The technical field generally relates to a dilutable solid foaming cleanser composition, and in particular to a dilutable solid foaming cleanser composition for use as a handwash.

### BACKGROUND

There is an increasing desire to reduce packaging waste as well as energy costs in production and transportation. Typically, cleansing compositions, such as handwashes, are sold as individually packaged liquids. Each individual package is intended for a single use and is disposable. Further, because each individual package is filled with a ready-to-use aqueous solution, shipping weight and costs are relatively high. Also, storage space is taken up by packaging that might contain over 80% water.

Therefore, forming an anhydrous cleansing composition to which water may be added after production, shipping, sale, and purchase provides for a reduction in manufacturing costs, packaging costs, shipping costs and environmental impact. However, performance of the cleansing solution is still vitally important to the consumer.

Accordingly, it is desirable to provide a solid concentrated composition for forming a foaming cleanser solution, such as for use as a handwash. Also, it is desirable to provide methods for forming such a solid concentrated composition, as well as methods for forming a foaming cleanser solution from such a solid concentrated composition. Furthermore, other desirable features and characteristics will become apparent from the subsequent detailed description and the appended claims, taken in conjunction with this background.

### BRIEF SUMMARY

Solid concentrated compositions of foaming cleansers, methods for preparing such solid concentrated compositions, and methods for forming liquid cleansing solutions from such solid concentrated compositions are provided. An exemplary solid concentrated composition includes a surfactant and a dissolution promotion agent, wherein the composition is anhydrous, and wherein the composition is substantially free of sulfates.

In another embodiment, a method for preparing a solid concentrated composition of a foaming cleanser is provided. The exemplary method includes blending homogeneously a humectant, a surfactant, and a dissolution promotion agent to form a mixture. Further, the method includes compressing the mixture to form the solid concentrated composition. In the exemplary method, the solid concentrated composition is anhydrous, and the solid concentrated composition is substantially free of sulfates.

In yet another embodiment, a method for forming a liquid cleansing solution is provided. The method includes dissolving a solid concentrated composition of a foaming cleanser in water to form a transparent solution. In the method, the solid concentrated composition is formulated to completely dissolve in water at ambient temperature and pressure in less than 90 minutes with no added agitation. Further, the solid concentrated composition comprises a humectant, a surfactant, and a dissolution promotion agent, the solid concentrated composition is anhydrous, and the solid concentrated composition is substantially free of sulfates.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### DETAILED DESCRIPTION

The following detailed description is merely illustrative in nature and is not intended to limit the embodiments of the subject matter or the application and uses of such embodiments. As used herein, the word "exemplary" means "serving as an example, instance, or illustration." Any implementation described herein as exemplary is not necessarily to be construed as preferred or advantageous over other implementations. Furthermore, there is no intention to be bound by any expressed or implied theory presented in the preceding technical field, background, brief summary or the following detailed description.

As used herein, "a," "an," or "the" means one or more unless otherwise specified. The term "or" can be conjunctive or disjunctive. Open terms such as "include," "including," "contain," "containing" and the like mean "comprising." The term "about" as used in connection with a numerical value denotes an interval of accuracy, familiar and acceptable to a person skilled in the art. In general, such interval of accuracy is ± ten percent. Thus, "about ten" means nine to eleven. In certain embodiments, the term "about" as used in connection with a numerical value may denote that an actual value may be rounded to the numerical value. For example, "about 1" may mean a value from 0.5 to 1.49. All numbers in this description indicating amounts, ratios of materials, physical properties of materials, and/or use are to be understood as modified by the word "about," except as otherwise explicitly indicated. As used herein, the "%" described in the present disclosure refers to the weight percentage (wt.%) unless otherwise indicated, and all weight percentages are based on the total weight of the solid composition unless defined otherwise.

Exemplary concentrated compositions of foaming cleansers are substantially free of sulfates. The term "substantially free of" as utilized herein means that the composition may include insignificant amounts of sulfates such that the composition includes no greater than 0.5 wt%, alternatively no greaterthan 0.1 wt%, or alternatively no greaterthan 0.05 wt% or no greaterthan 0.01 wt%. In certain embodiments, the concentrated compositions of foaming cleansers are completely free of sulfates, such that no measurable amount of sulfate is present in the compositions.

By avoiding use of sulfates, foaming cleansers formed from the solid compositions herein are less harsh than sulfate cleansers. However, compositions using sulfates generally foam well and have good solubility. As described herein, concentrated compositions of foaming cleansers are provided with good foaming properties, increased water solubility, i.e., quick dissolution, and appropriate hand feel, despite being sulfate free.

Embodiments described in detail below include anhydrous solid forms of concentrated compositions of foaming cleansers. Such forms may include tablets, pods, powder, or other desired solid forms. In exemplary embodiments, the foaming cleansers are handwashes. However, the foaming cleaners may be provided for other suitable cleansing uses, for example in personal care products including bodywash.

It is contemplated that the described solid forms of concentrated compositions of foaming cleansers be prepared, shipped, sold, and purchased in the anhydrous form. For use, a purchaser may dissolve a unit of the concentrated composition in water in a non-disposable container. As a result, use of disposable containers is reduced, and production and shipping costs are decreased.

The term "anhydrous" as used herein refers to a composition comprising less than 2 wt.%, such as less than 1 wt.%, for example less than 0.5 wt.%, such as less than 0.1 wt.%, for example less than 0.01 wt.%, or less than 0.001 wt.% of water based on the total weight of the composition.

An exemplary solid concentrated composition may be dissolved in water at a composition:water weight ratio of 1:25 to form a cleansing solution. At such a ratio, the solid concentrated composition fully dissolves in water in less than 90 minutes, such as in less than 60 minutes, for example in less than 40 minutes, at ambient pressure and temperature, for example at a temperature of 25 °C and pressure of 1 ATM. When fully dissolved, the resulting solution is completely clear or transparent. This is unlike current commercial products which remain cloudy in solution and/or are not fully dissolved in water.

Because exemplary solid concentrated compositions described herein fully dissolve in water to form a completely clear or transparent solution, a visually/aesthetically pleasing effect is provided. Further, by attaining a clear visibility, the user is ensured that all active ingredients in the solid concentrated composition are dispersed evenly throughout the solution. When current commercial products remain cloudy there is an indication of, or at least possibility of, incomplete dissolution as well as uneven dispersion of components in the solvent. Further, incomplete dissolution may result in settling of components such that an upper portion of a solution may have a lower concentration of solute (and higher concentration of solvent) while a lower portion of a solution may have a higher concentration of solute (and lower concentration of solvent). This could result in poor cleansing when a user dispenses cleanser from an upper portion of a full solution. Further, this could result in skin irritation due to too much cleansing when a more concentrated portion of the cleanser is dispensed from the lower remaining portion of solution.

Further, in exemplary embodiments, the cleansing solution prepared from the exemplary solid concentrated composition dissolved in water is clear, i.e., fully transparent. Clarity of the cleansing solutions described herein can be determined in relation to the turbidity or transparency of the composition according to DIN/EN 27027 (ISO 7027) as a transmitted light turbidity measurement. As the result, the transparency is obtained as a percentage of transmittance. Transmittance is the percentage of incident light that is transmitted, i.e., successfully passes through the composition. In certain embodiments, a TurbiScan MA 2000 (measuring instrument from Formulaction) may be utilized to measure transmission at 23°C. For exemplary cleansing solutions described herein, the percentage of transmittance as measured according to DIN/EN 27027 (ISO 7027) is at least 50%, such as at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

### SURFACTANT

In exemplary embodiments, the solid concentrated composition includes a surfactant. An exemplary surfactant includes a primary surfactant and a secondary surfactant. In certain embodiments, the surfactant consists of a primary surfactant and a secondary surfactant. In certain embodiments, the primary surfactant may include more than one primary surfactant compounds and the secondary surfactant may include more than one secondary surfactant compound. In certain embodiments, the primary surfactant may consist of one primary surfactant compound and the secondary surfactant may consist of one secondary surfactant compound.

In a particular exemplary embodiment, the surfactant forms 28.2 wt.% of the composition. In exemplary embodiments, the surfactant forms at least 5 wt.% of the composition, such as at least 10 wt.% of the composition, for example at least 15 wt.% of the composition, such as at least 20 wt.% of the composition, for example at least 22 wt.% of the composition, such as at least 24 wt.% of the composition, for example at least 25 wt.% of the composition, such as at least 26 wt.% of the composition, for example at least 27 wt.% of the composition, such as at least 28 wt.% of the composition, for example at least 29 wt.% of the composition, such as at least 30 wt.% of the composition, for example at least 31 wt.% of the composition, such as at least 32 wt.% of the composition, for example at least 33 wt.% of the composition or at least 35 wt.% of the composition. In exemplary embodiments, the surfactant forms no more than 52 wt.% of the composition, such as no more than 50 wt.% of the composition, for example no more than 45 wt.% of the composition, such as no more than 40 wt.% of the composition, for example no more than 35 wt.% of the composition, such as no more than 34 wt.% of the composition, for example no more than 33 wt.% of the composition, such as no more than 32 wt.% of the composition, for example no more than 31 wt.% of the composition, such as no more than 30 wt.% of the composition, for example no more than 29 wt.% of the composition, such as no more than 28 wt.% of the composition, for example no more than 27 wt.% of the composition, such as no more than 26 wt.% of the composition, for example no more than 25 wt.% of the composition

### PRIMARY SURFACTANT

An exemplary primary surfactant is suitable for removing soil, and in particular for removing soil from skin such as from a user's hands. In exemplary embodiments, the primary surfactant may comprise more than one surfactant. An exemplary primary surfactant consists of a single surfactant. In an exemplary embodiment, the primary surfactant is a non-sulfate surfactant. Further, in an exemplary embodiment, the primary surfactant is an anionic surfactant. In other embodiments, the primary surfactant may be selected from anionic, non-ionic, cationic, and amphoteric (zwitterionic) surfactants, or combinations thereof.

In an exemplary embodiment, the primary surfactant is a taurate. In a particular exemplary embodiment, the primary surfactant is sodium methyl cocoyl taurate. Other suitable primary surfactants include isethionates, such as sodium cocoyl isethionate, and glutamates, such as disodium cocoyl glutamate or sodium cocoyl glutamate.

In a particular exemplary embodiment, the primary surfactant forms 27 wt.% of the composition. In exemplary embodiments, the primary surfactant forms at least 5 wt.% of the composition, such as at least 10 wt.% of the composition, for example at least 15 wt.% of the composition, such as at least 17.5 wt.% of the composition, for example at least 20 wt.% of the composition, such as at least 21 wt.% of the composition, for example at least 22 wt.% of the composition, such as at least 23 wt.% of the composition, for example at least 24 wt.% of the composition, such as at least 25 wt.% of the composition, for example at least 26 wt.% of the composition, such as at least 27 wt.% of the composition, for example at least 28 wt.% of the composition or at least 30 wt.% of the composition. In exemplary embodiments, the primary surfactant forms no more than 50 wt.% of the composition, such as no more than 45 wt.% of the composition, for example no more than 40 wt.% of the composition, such as no more than 35 wt.% of the composition, for example no more than 33 wt.% of the composition, such as no more than 32 wt.% of the composition, for example no more than 31 wt.% of the composition, such as no more than 30 wt.% of the composition, for example no more than 29 wt.% of the composition, such as no more than 28 wt.% of the composition, for example no more than 27 wt.% of the composition, such as no more than 25 wt.% of the composition or for example no more than 20 wt.% of the composition.

### SECONDARY SURFACTANT

An exemplary secondary surfactant is a co-surfactant suitable for use in enhancing the effectiveness of the selected primary surfactant. In exemplary embodiments, the secondary surfactant may comprise more than one surfactant. An exemplary secondary surfactant consists of a single surfactant. In an exemplary embodiment, the secondary surfactant is a non-sulfate surfactant. Further, in an exemplary embodiment, the secondary surfactant is an amphoteric (zwitterionic) surfactant. In other embodiments, the secondary surfactant may be selected from amphoteric (zwitterionic), anionic, non-ionic, and cationic surfactants, or combinations thereof.

In an exemplary embodiment, the secondary surfactant is a fatty acid amide. In a particular exemplary embodiment, the secondary surfactant is cocamidopropyl betaine.

In a particular exemplary embodiment, the secondary surfactant forms 1.20 wt.% of the composition. In exemplary embodiments, the primary surfactant forms at least 0.1 wt.% of the composition, such as at least 0.2 wt.% of the composition, for example at least 0.3 wt.% of the composition, such as at least 0.4 wt.% of the composition, for example at least 0.5 wt.% of the composition, such as at least 0.6 wt.% of the composition, for example at least 0.7 wt.% of the composition, such as at least 0.8 wt.% of the composition, for example at least 0.9 wt.% of the composition, such as at least 1.0 wt.% of the composition, for example at least 1.1 wt.% of the composition, such as at least 1.2 wt.% of the composition, for example at least 1.5 wt.% of the composition, or at least 2 wt.% of the composition.

In exemplary embodiments, the secondary surfactant forms no more than 10 wt.% of the composition, such as no more than 8 wt.% of the composition, for example no more than 6 wt.% of the composition, such as no more than 5 wt.% of the composition, for example no more than 4 wt.% of the composition, such as no more than 3 wt.% of the composition, for example no more than 2 wt.% of the composition, such as no more than 1.9 wt.% of the composition, for example no more than 1.8 wt.% of the composition, such as no more than 1.7 wt.% of the composition, for example no more than 1.6 wt.% of the composition, such as no more than 1.5 wt.% of the composition, for example no more than 1.4 wt.% of the composition, such as no more than 1.3 wt.% of the composition, for example no more than 1.2 wt.% of the composition, such as no more than 1.1 wt.% of the composition, or no more than 1.0 wt.% of the composition.

### DISSOLUTION PROMOTION AGENT

In exemplary embodiments, the composition includes a dissolution promotion agent that enhances, i.e., quickens, dissolution of the solid composition in a solvent such as water. An exemplary dissolution promotion agent includes a first dissolution promotion agent and a second dissolution promotion agent. In certain embodiments, the dissolution promotion agent consists of a first dissolution promotion agent and a second dissolution promotion agent. In certain embodiments, the dissolution promotion agents may be provided as pH adjustment agents to control the pH of the solid composition and/or of the final liquid solution in which the solid composition is dissolved. For example, the first dissolution promotion agent may be an acidic pH adjustment agent, i.e., an acidic dissolution promotion agent, and the second dissolution promotion agent may be a basic pH adjustment agent, i.e., a basic dissolution promotion agent. In other embodiments, the pH adjustment agents may be independent of, and additional to, the dissolution promotion agents.

In certain embodiments, the dissolution promotion agent or agents may enhance dissolution of the solid composition in a solvent. For example, a dissolution promotion agent may react with another dissolution promotion agent or with other components to produce a gaseous product, or effervescence, causing physical break-up of the solid. Further, formation of a gaseous product within the liquid solvent causes movement of the surrounding solvent to accelerate dissolution of the solid therein.

In a particular exemplary embodiment, the dissolution promotion agent forms at least 50.0 wt.% of the composition. In exemplary embodiments, the dissolution promotion agent forms at least 25 wt.% of the composition, such as at least 30 wt.% of the composition, for example at least 35 wt.% of the composition, such as at least 40 wt.% of the composition, for example at least 42 wt.% of the composition, such as at least 44 wt.% of the composition, for example at least 45 wt.% of the composition, such as at least 46 wt.% of the composition, for example at least 47 wt.% of the composition, such as at least 48 wt.% of the composition, for example at least 49 wt.% of the composition, such as at least 50 wt.% of the composition, for example at least 51 wt.% of the composition, such as at least 52 wt.% of the composition, for example at least 53 wt.% of the composition or at least 55 wt.% of the composition. In exemplary embodiments, the dissolution promotion agent forms no more than 75 wt.% of the composition, such as no more than 70 wt.% of the composition, for example no more than 65 wt.% of the composition, such as no more than 60 wt.% of the composition, for example no more than 55 wt.% of the composition, such as no more than 54 wt.% of the composition, for example no more than 53 wt.% of the composition, such as no more than 52 wt.% of the composition, for example no more than 51 wt.% of the composition, such as no more than 50 wt.% of the composition, for example no more than 49 wt.% of the composition, such as no more than 48 wt.% of the composition, for example no more than 27 wt.% of the composition, such as no more than 26 wt.% of the composition, for example no more than 45 wt.% of the composition.

### ACIDIC DISSOLUTION PROMOTION AGENT

As described above, an acidic dissolution promotion agent may be provided to react with another dissolution promotion agent, such as a basic dissolution promotion agent, or with other components to produce a gaseous product, or effervescence, causing physical break-up of the solid. Further, the acidic dissolution promotion agent may be used as a pH modifier in the solution formed with the solid dissolves. An exemplary acidic dissolution promotion agent has a pH of from 3 to 6, such as from 3 to 5, for example from 3 to 4, or from 3 to 3.5. In exemplary embodiments, the acidic dissolution promotion agent is an organic acid. For example, the acidic dissolution promotion agent may be citric acid and/or malic acid. An exemplary acidic dissolution promotion agent consists of citric acid.

In a particular exemplary embodiment, the acidic dissolution promotion agent forms 30 wt.% of the composition. In exemplary embodiments, the acidic dissolution promotion agent forms at least 10 wt.% of the composition, such as at least 15 wt.% of the composition, for example at least 20 wt.% of the composition, such as at least 22 wt.% of the composition, for example at least 24 wt.% of the composition, such as at least 25 wt.% of the composition, for example at least 26 wt.% of the composition, such as at least 27 wt.% of the composition, for example at least 28 wt.% of the composition, such as at least 29 wt.% of the composition, for example at least 30 wt.% of the composition, such as at least 31 wt.% of the composition, for example at least 32 wt.% of the composition, or at least 33 wt.% of the composition.

In exemplary embodiments, the acidic dissolution promotion agent forms no more than 50 wt.% of the composition, such as no more than 45 wt.% of the composition, for example no more than 40 wt.% of the composition, such as no more than 38 wt.% of the composition, for example no more than 36 wt.% of the composition, such as no more than 35 wt.% of the composition, for example no more than 34 wt.% of the composition, such as no more than 33 wt.% of the composition, for example no more than 32 wt.% of the composition, such as no more than 31 wt.% of the composition, for example no more than 30 wt.% of the composition, such as no more than 29 wt.% of the composition, for example no more than 28 wt.% of the composition.

### BASIC DISSOLUTION PROMOTION AGENT

As described above, a basic dissolution promotion agent may be provided to react with another dissolution promotion agent, such as an acidic dissolution promotion agent, or with other components to produce a gaseous product, or effervescence, causing physical break-up of the solid. Further, the basic dissolution promotion agent may be used as a pH modifier in the solution formed with the solid dissolves. An exemplary basic dissolution promotion agent has a pH of from 8 to 12, such as from 8 to 9, 9 to 10, 10 to 11, or 11 to 12. In exemplary embodiments, the basic dissolution promotion agent is a carbonate, such as sodium carbonate, sodium bicarbonate, or other suitable alkali carbonates. An exemplary acidic dissolution promotion agent consists of sodium bicarbonate.

In a particular exemplary embodiment, the basic dissolution promotion agent forms 20 wt.% of the composition. In exemplary embodiments, the basic dissolution promotion agent forms at least 5 wt.% of the composition, for example at least 7.5 wt.% of the composition, such as at least 10 wt.% of the composition, for example at least 12.5 wt.% of the composition, such as at least 15 wt.% of the composition, for example at least 16 wt.% of the composition, such as at least 17 wt.% of the composition, for example at least 18 wt.% of the composition, such as at least 19 wt.% of the composition, for example at least 20 wt.% of the composition, such as at least 21 wt.% of the composition, for example at least 22 wt.% of the composition, such as at least 25 wt.% of the composition.

In exemplary embodiments, the acidic dissolution promotion agent forms no more than 40 wt.% of the composition, such as no more than 35 wt.% of the composition, for example no more than 30 wt.% of the composition, such as no more than 28 wt.% of the composition, for example no more than 26 wt.% of the composition, such as no more than 25 wt.% of the composition, for example no more than 24 wt.% of the composition, such as no more than 23 wt.% of the composition, for example no more than 22 wt.% of the composition, such as no more than 21 wt.% of the composition, for example no more than 20 wt.% of the composition, such as no more than 19 wt.% of the composition, for example no more than 18 wt.% of the composition.

### HUMECTANT

In exemplary embodiments, the solid concentrated composition includes a humectant. An exemplary humectants may provide for enhanced dissolution of the solid composition in water by increasing the rate of water uptake. Humectants are hygroscopic generally because of the abundance of hydroxyl groups on the molecule which will react with water. In an exemplary embodiment, the humectant comprises sorbitol. Sorbitol has six hydroxyl groups which facilitates quick dissolution. An exemplary humectant consists of sorbitol. Other suitable humectants may include glycerin, sodium PCA, sodium lactate, alpha hydroxy acids such as lactic acid, and other sugar alcohols such as xylitol or maltitol, and combinations thereof or in combination with sorbitol.

In a particular exemplary embodiment, the humectant forms 9.4 wt.% of the composition. In exemplary embodiments, the humectant forms at least 4 wt.% of the composition, such as at least 5 wt.% of the composition, for example at least 6 wt.% of the composition, such as at least 7 wt.% of the composition, for example at least 8 wt.% of the composition, such as at least 9 wt.% of the composition, for example at least 10 wt.% of the composition, such as at least 11 wt.% of the composition, for example at least 12 wt.% of the composition. In exemplary embodiments, the humectant forms no more than 15 wt.% of the composition, such as no more than 14 wt.% of the composition, for example no more than 13 wt.% of the composition, such as no more than 12 wt.% of the composition, for example no more than 11 wt.% of the composition, such as no more than 10 wt.% of the composition, for example no more than 9 wt.% of the composition, such as no more than 8 wt.% of the composition, for example no more than 7 wt.% of the composition, such as no more than 6 wt.% of the composition.

### PRESERVATIVE

In exemplary embodiments, the solid concentrated composition includes a preservative. In an exemplary embodiment, the preservative comprises sodium benzoate. An exemplary preservative consists of sodium benzoate as sodium benzoate. Suitable preservatives that are accepted by consumers include organic acids such as benzoic acid, sorbic acid, levulinic acid, among others. Parabens may be effective as preservative but have poor acceptance by consumers.

In a particular exemplary embodiment, the preservative forms 3.1 wt.% of the composition. In exemplary embodiments, the preservative forms at least 1 wt.% of the composition, such as at least 1.5 wt.% of the composition, for example at least 2 wt.% of the composition, such as at least 2.5 wt.% of the composition, for example at least 2.6 wt.% of the composition, such as at least 2.7 wt.% of the composition, for example at least 2.8 wt.% of the composition, such as at least 2.9 wt.% of the composition, for example at least 3.0 wt.% of the composition, such as at least 3.1 wt.% of the composition, for example at least 3.2 wt.% of the composition. In exemplary embodiments, the preservative forms no more than 5 wt.% of the composition, such as no more than 4.5 wt.% of the composition, for example no more than 4 wt.% of the composition, such as no more than 3.5 wt.% of the composition, for example no more than 3.4 wt.% of the composition, such as no more than 3.3 wt.% of the composition, for example no more than 3.2 wt.% of the composition, such as no more than 3.1 wt.% of the composition, for example no more than 3 wt.% of the composition, such as no more than 2.9 wt.% of the composition.

### EMOLLIENT

In exemplary embodiments, the solid concentrated composition includes an emollient. A suitable emollient or moisturizer provides the user with appropriate hand feel. In an exemplary embodiment, the emollient comprises allantoin. An exemplary emollient consists of allantoin. Other suitable emollients include butters, esters, natural ingredients such as silk protein powder, colloidal oatmeal, different plant extracts, carrageenan, guar gum, and the like.

In a particular exemplary embodiment, the emollient forms 3.1 wt.% of the composition. In exemplary embodiments, the emollient forms at least 1 wt.% of the composition, such as at least 1.5 wt.% of the composition, for example at least 2 wt.% of the composition, such as at least 2.5 wt.% of the composition, for example at least 2.6 wt.% of the composition, such as at least 2.7 wt.% of the composition, for example at least 2.8 wt.% of the composition, such as at least 2.9 wt.% of the composition, for example at least 3.0 wt.% of the composition, such as at least 3.1 wt.% of the composition, for example at least 3.2 wt.% of the composition. In exemplary embodiments, the emollient forms no more than 5 wt.% of the composition, such as no more than 4.5 wt.% of the composition, for example no more than 4 wt.% of the composition, such as no more than 3.5 wt.% of the composition, for example no more than 3.4 wt.% of the composition, such as no more than 3.3 wt.% of the composition, for example no more than 3.2 wt.% of the composition, such as no more than 3.1 wt.% of the composition, for example no more than 3 wt.% of the composition, such as no more than 2.9 wt.% of the composition.

### FRAGRANCE

In exemplary embodiments, the solid concentrated composition includes a fragrance. In a particular exemplary embodiment, the fragrance forms 6.2 wt.% of the composition. In exemplary embodiments, the fragrance forms at least 3 wt.% of the composition, such as at least 3.5 wt.% of the composition, for example at least 4 wt.% of the composition, such as at least 4.5 wt.% of the composition, for example at least 5 wt.% of the composition, such as at least 5.5 wt.% of the composition, for example at least 6 wt.% of the composition, such as at least 6.1 wt.% of the composition, for example at least 6.2 wt.% of the composition, such as at least 6.4 wt.% of the composition, for example at least 6.5 wt.% of the composition. In exemplary embodiments, the fragrance forms no more than 8 wt.% of the composition, such as no more than 7.5 wt.% of the composition, for example no more than 7 wt.% of the composition, such as no more than 6.5 wt.% of the composition, for example no more than 6.4 wt.% of the composition, such as no more than 6.3 wt.% of the composition, for example no more than 6.2 wt.% of the composition, such as no more than 6.1 wt.% of the composition, for example no more than 6 wt.% of the composition, such as no more than 5.9 wt.% of the composition.

### OTHER COMPONENTS

In certain embodiments, the solid concentrated composition may include other components in addition to those described above. For example, the solid concentrated composition may include preservative booster, a water softening agent, a viscosity adjuster, a thickening agent, a chelating agent, a lubricating agent, and a binding agent.

In certain embodiments, the solid concentrated composition consists of, or consists essentially of a surfactant, a dissolution promotion agent, and a humectants, and optionally of a preservative, an emollient, and/or fragrance.

### EXAMPLE

Table I provides a breakdown of five samples, including two comparison samples using sulfates as a primary surfactant (Samples A and B), as well as Sample C using Sodium Cocoyl Isethionate (and) Disodium Cocoyl Glutamate, Sample D using Sodium Cocoyl Glutamate, and Sample E using Sodium Methyl Cocoyl Taurate. The samples were formed into 8g tablets by combining the ingredients as set forth in Table 1 via the method described herein. The compressed 8g tablets were then dissolved in 200g water with no agitation applied.

| TABLE I | Ingredient | Sample | | | | |
|---|---|---|---|---|---|---|
| | | A | B | C | D | E |
| Primary Surfactant | Sodium Coco Sulfate | 27 | - | - | - | - |
| | Sodium Lauryl Sulfate | - | 27 | - | - | - |
| | Sodium Cocoyl Isethionate (and) Disodium Cocoyl Glutamate | - | - | 27 | - | - |
| | Sodium Cocoyl Glutamate | - | - | - | 27 | |
| | Sodium Methyl Cocoyl Taurate | - | - | - | - | 27 |
| Secondary Surfactant | Cocamidopropyl Betaine | 1.20 | 1.20 | 1.20 | 1.20 | 1.20 |
| Acid pH Adjuster / Dissolution Agent | Citric Acid | 30.00 | 30.00 | 30.00 | 30.00 | 30.00 |
| Base pH Adjuster / Dissolution Agent | Sodium Bicarbonate | 20.00 | 20.00 | 20.00 | 20.00 | 20.00 |
| Humectant | Sorbitol | 9.40 | 9.40 | 9.40 | 9.40 | 9.40 |
| Preservative | Sodium Benzoate | 3.10 | 3.10 | 3.10 | 3.10 | 3.10 |
| Emollient | Allantoin | 3.10 | 3.10 | 3.10 | 3.10 | 3.10 |
| Fraqrance | Fragrance | 6.20 | 6.20 | 6.20 | 6.20 | 6.20 |
| TOTAL wt.% | | 100 | 100 | 100 | 100 | 100 |

Testing was performed on each of the solid concentrated compositions of Samples A-E. Testing on cleansing solutions formed from the samples revealed that the sulfate-containing Samples A and B both exhibited skin irritation while the sulfate-free Samples C, D and E were less irritating. Further, Samples A and B exhibited poor clarity, i.e., the cleansing solution formed from the solid concentrated composition was not clear, while Sample E had very good clarity, and was clear. Also, while the time needed to dissolve Sample A in 25 °C water was over three hours, Sample E was dissolved in 25 °C water in about forty minutes, under the same conditions.

A method for preparing a solid concentrated composition of a foaming cleanser is also contemplated herein. The exemplary method includes blending homogeneously a humectant, a surfactant, and a dissolution promotion agent to form a mixture. During blending a preservative, an emollient, and fragrance may be blended with the humectant, the surfactant, and the dissolution promotion agent to form the mixture. Further, the method includes compressing the mixture to form the solid concentrated composition. In such a method, the solid concentrated composition is anhydrous, and the solid concentrated composition is substantially free of sulfates.

Also provided is a method for forming a liquid cleansing solution. Such a method includes dissolving a solid concentrated composition of a foaming cleanser in water to form a transparent solution. The solid concentrated composition is formulated to completely dissolve in water at a temperature of 25° C in less than 90 minutes with no added agitation. Further, the solid concentrated composition comprises a humectant, a surfactant, and a dissolution promotion agent. Also, the solid concentrated composition is anhydrous, and the solid concentrated composition is substantially free of sulfates. In the method, the solid concentrated composition is dissolved in water at a composition:water weight ratio of 1:25 to form the liquid cleansing solution.

As described herein, a solid concentrated composition is provided for dissolution in water to form a sulfate-free non-irritating, clear, foaming cleanser. The exemplary solid concentrated composition more quickly dissolves in water as compared to conventional sulfate-containing compositions.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration in any way. Rather, the foregoing detailed description will provide those skilled in the art with a convenient road map for implementing an exemplary embodiment. It being understood that various changes may be made in the function and arrangement of elements described in an exemplary embodiment without departing from the scope as set forth in the appended claims.

## Claims

1. A solid concentrated composition of a foaming cleanser, the composition comprising:
a surfactant; and
a dissolution promotion agent,
wherein the composition is anhydrous, and
wherein the composition is substantially free of sulfates.

2. The solid concentrated composition of claim 1, wherein:
the composition has a dissolved configuration wherein the composition is dissolved in water at a composition:water weight ratio of 1:25 to form a cleansing solution, and
when in the dissolved configuration, the cleansing solution is transparent.

3. The solid concentrated composition of claim 1, wherein the composition has a solubility in water such that the composition is completely dissolved in water in less than 90 minutes at ambient pressure and temperature, with no added agitation.

4. The solid concentrated composition of claim 1 wherein the surfactant comprises a primary surfactant and a secondary surfactant.

5. The solid concentrated composition of claim 1 wherein the surfactant comprises a primary surfactant and a secondary surfactant, and wherein the composition has a primary surfactant to secondary surfactant ratio of from about 20:1 to about 25:1.

6. The solid concentrated composition of claim 1 wherein the surfactant consists of a primary surfactant and a secondary surfactant.

7. The solid concentrated composition of claim 1 wherein the surfactant comprises a primary surfactant and a secondary surfactant, wherein the primary surfactant is an anionic surfactant, and wherein the secondary surfactant is an amphoteric surfactant.

8. The solid concentrated composition of claim 1 wherein the surfactant comprises a primary surfactant and a secondary surfactant, wherein the primary surfactant is a taurate, and wherein the secondary surfactant is a fatty acid amide.

9. The solid concentrated composition of claim 1 wherein the surfactant comprises a primary surfactant and a secondary surfactant, wherein the primary surfactant is sodium methyl cocoyl taurate, and wherein the secondary surfactant is cocamidopropyl betaine.

10. The solid concentrated composition of claim 1 wherein the composition consists of:
the surfactant;
the dissolution promotion agent,
a humectant; and
optionally, a preservative, an emollient, and/or fragrance.

11. The solid concentrated composition of claim 1 wherein:
the composition comprises:
from about 20 to about 40 wt.% of the surfactant; and
from about 30 to about 70 wt.% of the dissolution promotion agent,
all based on a total weight of the composition.

12. The solid concentrated composition of claim 1 further comprising a humectant, wherein the composition comprises:
from about 30 to about 70 wt.% of the dissolution promotion agent;
from about 20 to about 40 wt.% of the surfactant; and
from about 5 to about 15 wt.% of the humectant,
all based on a total weight of the composition.

13. The solid concentrated composition of claim 1 wherein:
the surfactant comprises a primary surfactant and a secondary surfactant;
the dissolution promotion agent comprises an acidic dissolution promotion agent and a basic dissolution promotion agent; and
the composition comprises:
from about 20 to about 35 wt.% primary surfactant;
from about 0.8 to about 1.6 wt.% secondary surfactant;
from about 20 to about 40 wt.% acidic dissolution promotion agent; and
from about 10 to about 30 wt.% basic dissolution promotion agent,
all based on a total weight of the composition.

14. The solid concentrated composition of claim 13 further comprising:
from about 1 to about 5 wt.% preservative;
from about 5 to about 15 wt.% humectant;
from about 1 to about 5 wt.% emollient; and
from about 2 to about 10 wt.% fragrance,
all based on a total weight of the composition.

15. The solid concentrated composition of claim 1 wherein the composition comprises:
about 28 wt.% of the surfactant;
about 50 wt.% of the dissolution promotion agent;
about 9 wt.% of humectant;
about 3 wt.% of preservative;
about 3 wt.% emollient; and
about 6 wt.% fragrance,
all based on a total weight of the composition.

16. The solid concentrated composition of claim 1 wherein the composition comprises:
about 27 wt.% of sodium methyl cocoyl taurate;
about 1 wt.% of cocamidopropyl betaine, wherein the surfactant is comprised of the sodium methyl cocoyl taurate and the cocamidopropyl betaine;
about 30 wt.% of citric acid;
about 40 wt.% of sodium bicarbonate, wherein the dissolution promotion agent is comprised of the citric acid and the sodium bicarbonate;
about 9 wt.% of sorbitol;
about 3 wt.% of sodium benzoate;
about 3 wt.% of allantoin; and
about 6 wt.% of fragrance,
all based on a total weight of the composition.

17. A method for preparing a solid concentrated composition of a foaming cleanser, the method comprising:
blending homogeneously a humectant, a surfactant, and a dissolution promotion agent to form a mixture; and
compressing the mixture to form the solid concentrated composition,
wherein the solid concentrated composition is anhydrous, and
wherein the solid concentrated composition is substantially free of sulfates.

18. The method of claim 17 further comprising blending homogeneously a preservative, emollient, and fragrance with the humectant, the surfactant, and the dissolution promotion agent to form the mixture.

19. A method for forming a liquid cleansing solution, the method comprising:
dissolving a solid concentrated composition of a foaming cleanser in water to form a transparent solution, wherein:
the solid concentrated composition is formulated to completely dissolve in water at ambient temperature and pressure in less than 90 minutes with no added agitation;
the solid concentrated composition comprises a humectant, a surfactant, and a dissolution promotion agent;
the solid concentrated composition is anhydrous; and
the solid concentrated composition is substantially free of sulfates.

20. The method of claim 19 wherein the solid concentrated composition is dissolved in water at a composition:water weight ratio of 1:25 to form the liquid cleansing solution.
